## Europäisches Patentamt

## European Patent Office

## Office européen des brevets

(19)

(11) Veröffentlichungsnummer: **0 175 007**

**B1**

(12) **EUROPÄISCHE PATENTSCHRIFT**

(45) Veröffentlichungstag der Patentschrift:
**27.04.88**

(51) Int. Cl.⁴: **C 12 P 7/46**

(21) Anmeldenummer: **84111098.4**

(22) Anmeldetag: **18.09.84**

---

(54) **Verfahren zur biotechnischen Herstellung von L-Äpfelsäure.**

---

(43) Veröffentlichungstag der Anmeldung:
**26.03.86 Patentblatt 86/13**

(45) Bekanntmachung des Hinweises auf die Patenterteilung:
**27.04.88 Patentblatt 88/17**

(84) Benannte Vertragsstaaten:
**AT BE CH DE FR GB IT LI LU NL SE**

(56) Entgegenhaltungen:
**EP-A-0 115 606**
**US-A-3 922 195**

(73) Patentinhaber: **HÜLS AKTIENGESELLSCHAFT, Patentabteilung / PB 15 - Postfach 13 20, D-4370 Marl 1 (DE)**

(72) Erfinder: **Schindler, Fritz, Dr., Am Stadtgarten 1/1959, D-4650 Gelsenkirchen (DE)**

---

**Beschreibung**

Die Erfindung betrifft ein Verfahren zum Herstellen des reinen L-Isomeren der Äpfelsäure aus neutralisierter Fumarsäure in hochkonzentrierter wäßriger Lösung durch biotechnische Umsetzung und einer Konzentration der L-Äpfelsäure zum Zeitpunkt der Ernte von 170 bis 400 g pro Liter der Fermentationsflüssigkeit, in der die L-Äpfelsäure in Form eines Salzes vorliegt.

L-Äpfelsäure wird in der Lebensmittel- und pharmazeutischen Industrie als Puffersubstanz, Komplexbildner, Acidulans und Feuchthaltemittel eingesetzt. L-Äpfelsäure ist als Naturstoff für diese Anwendungsgebiete geeigneter als das chemisch, z. B. durch Wasseranlagerung an Maleinsäureanhydrid, zugängliche D,L-Isomerengemisch der Äpfelsäure. Das D-Isomer der Äpfelsäure kommt in der Natur nicht vor, weshalb die Anwendung der chemisch synthetisierten D,L-Äpfelsäure im Lebensmittel- und Pharmabereich nicht unbedenklich ist.

Zweck der vorliegenden Erfindung ist die Verbesserung der Wirtschaftlichkeit der biotechnischen Herstellung von L-Äpfelsäure in reiner Form.

Zur biotechnischen Herstellung von L-Äpfelsäure sind mehrere Verfahren bekannt. Ein Teil dieser Verfahren (z. B. DE-AS-23 63 285) arbeitet mit speziellen Bakterien, die Fumarsäure durch Anlagerung von Wasser in L-Äpfelsäure überführen. In der US-3 922 195 wird vorgeschlagen, die Bakterienzellen zu immobilisieren. Nach DE-AS-23 63 285 wird mit freien Bakterienzellen und nach DE-AS-24 15 310 mit dem aus Bakterienzellen isolierten Enzym Fumarase gearbeitet.

Weiter ist die Umsetzung von Glucose in L-Äpfelsäure durch Vergesellschaftung eines Pilzes mit einem Bakterium beschrieben worden (J. Ferment. Technol., Vol. 54, No. 4 (1976), Seiten 197 bis 204). Hierbei bildet der Pilz aus Glucose Fumarsäure, die dann von dem Bakterium in L-Äpfelsäure überführt wird.

Das die Umsetzung von Fumarsäure in L-Äpfelsäure bewirkende Enzym, die Fumarase, kann aus biologischem Material, wie der Zellmasse von Bakterien oder Pilzen, in speziellen Verfahren isoliert und dann - in freier oder immobilisierter Form - zur Gewinnung von L-Äpfelsäure aus Fumarsäure eingesetzt werden (DE-AS-24 15 310; CS-171 990).

Bei einigen Verfahren wird dem Fermentationsansatz eine große Menge einer Kalzium-Verbindung zugegeben, wodurch die gebildete L-Äpfelsäure bereits während der Fermentation als Ca-Malat ausfällt (DE-OS-14 17 033).

Verfahren zur biotechnischen Herstellung von L-Äpfelsäure mit Hilfe von Pilzen sind ebenfalls bekannt. Bei diesen Verfahren wird die L-Äpfelsäure im wesentlichen durch biochemischen Ab- bzw. Umbau der an den betreffenden Pilz verfütterten Kohlenstoffquelle (z. B. Melasse, Zucker, Ethanol, Essigsäure) erhalten (J. Ferment. Technol. Vol. 55, No. 2 (1977), Seiten 196 bis 199). Diese Verfahren gehen nicht direkt von Fumarsäure aus.

Bei dem in US-3 063 910 beschriebenen Verfahren kann der Fermentationsansatz neben 10 bis 15 % eines Zuckers noch 1 bis 10 % einer organischen Säure, wie z. B. Brenztraubensäure oder Fumarsäure, enthalten. Jedoch dient bei diesem Pilz-Verfahren die zugesetzte organische Säure nicht als Substrat für die Synthese von L-Äpfelsäure, sondern als Reaktionsbeschleuniger des biochemischen Ab- bzw. Umbaus des angebotenen Zuckers in L-Äpfelsäure.

Weiter ist die fermentative Überführung von n-Paraffin in L-Äpfelsäure durch Vergesellschaftung von zwei Hefearten möglich, wobei die eine Hefeart Paraffin in Fumarsäure überführt und die zweite Hefeart die entstandene Fumarsäure in L-Äpfelsäure umwandelt (Agr. Biol. Chem., Vol. 34 (1970), Seiten 1 833 bis 1 838).

In US-4 013 508 wird die biotechnische Herstellung von L-Asparaginsäure aus Kohlenwasserstoffen mit Ammonium-Fumarat als Zwischenstufe beschrieben, wobei ein mit einem Pilz vergesellschaftetes Bakterium eingesetzt wird. Das Ammonium-Fumarat wird praktisch vollständig in L-Asparaginsäure überführt, wobei letztlich thermodynamische Gründe für die Bildung der L-Asparaginsäure und gegen die Bildung der L-Äpfelsäure aus Ammonium-Fumarat angeführt werden.

Weitere Angaben über die biotechnische Umwandlung von Ammonium-Fumarat in L-Asparaginsäure sind in DE-PS-23 45 271 und BE-818 480 zu finden.

Die bekannten Verfahren zur biotechnischen Herstellung von L-Äpfelsäure haben u. a. die nachstehend aufgeführten Eigenarten:

- Bei der Verwendung von Bakterien sind biotechnische Verfahren schwieriger zu handhaben als bei Verwendung von Pilzen. Aus der geringen Größe der Bakterien können beispielsweise Abtrennprobleme entstehen. Bakterien neigen ferner dazu, Nebenprodukte, darunter auch toxische Nebenprodukte, zu bilden. Besonders störend für eine Anwendung der L-Äpfelsäure im Pharmabereich ist das von Bakterien gebildete LPS-Toxin (Lipopolysaccharid-Toxin), das pyrogen wirkt, und von dem die L-Äpfelsäure nur durch aufwendige Verfahrensschritte, wie z. B. durch Ultrafiltration, zu reinigen ist. Bei dem in US-3 922 195 beschriebenen Bakterien-Verfahren tritt als Nebenprodukt in geringer Menge Bernsteinsäure auf, die von Äpfelsäure nur mit Schwierigkeiten abzutrennen ist.

- Beim Einsatz des Enzyms Fumarase kommt als Fumarase-Quelle in erster Linie mikrobielle Zellmasse infrage. Vor Beginn der Herstellung von L-Äpfelsäure muß mikrobielle Zellmasse gezüchtet und mit erheblichem Aufwind zu Fumarase aufgearbeitet werden.

- Die Immobilisierung von lebenden Zellen oder des aus Zellen isolierten Enzyms Fumarase ist ein zusätzlicher Verfahrensschritt.

- Bei der Fermentation in Gegenwart von Ca-Ionen in großer Konzentration wird die gebildete L-Äpfelsäure bereits während der Fermentation als Ca-Malat gefällt; das führt zu Problemen beim Rühren und Belüften der Fermentationsflüssigkeit.

- Die erzielbare Konzentration der L-Äpfelsäure ist im allgemeinen vergleichsweise gering und liegt deutlich unter 100 g pro Liter Fermentationsflüssigkeit, wenn man von den in US-3 922 195 (immobilisierte Bakterien) und in DE-OS-14 17 033 (hohe Kalziumkonzentration) beschriebenen Umsetzungen absieht.

- Die Fermentation dauert oft länger als drei Tage. Die wegen dieser geringen Produktivität erforderliche große Kapazität der Bio-Reaktoren verursacht einen großen apparativen Aufwand, zumal es sich um aufwendig gebaute Reaktoren handelt, die eine kontaminationsfreie Prozeßführung ermöglichen.

Obwohl der Begriff "Fermentation" in der Fachliteratur bisher einen relativ großen Bedeutungsumfang hat, wird in diesem Zusammenhang unter "Fermentation" die biochemische Umwandlung von Fumarsäure in L-Äpfelsäure unter Einwirkung der Fumarase verstanden. Mit "Züchtung" wird die Vermehrung der Biomasse aus dem Inoculum unter Verbrauch von Nährstoffen bezeichnet.

In DE-OS-33 10 849.8 wird die Herstellung des reinen L-Isomeren der Äpfelsäure aus neutralisierter Fumarsäure durch mikrobielle Fermentation mittels frei beweglicher Mikroorganismen in wäßriger Phase und einer Konzentration der L-Äpfelsäure zum Zeitpunkt der Ernte von 100 bis 170 g pro Liter der Fermentationsflüssigkeit beschrieben. Dieses Verfahren zeichnet sich aus durch eine große Produktivität. Die Ausbeute beträgt mehr als 77 % der theoretischen Ausbeute. Es entsteht keine D-Äpfelsäure. Die L-Äpfelsäure wird in einer Lösung erhalten, die deren Aufarbeitung zu einer für Nahrungsmittel und Pharmazeutika geeigneten Qualität sehr nahe kommt. Bei diesem Verfahren wird die Hauptmenge der Fumarsäure in Form ihres Natrium- und/oder Kalium-Salzes eingesetzt.

Obwohl dieses Verfahren über den Stand der Technik erheblich hinausgeht, stellt sich die Aufgabe, es weiter zu verbessern.

Diese Aufgabe wird erfindungsgemäß gelöst durch ein Verfahren mit folgenden kennzeichnenden Merkmalen:

- Die Menge der vorgelegten Fumarsäure beträgt 170 bis 400 g pro Liter des Ansatzes.
- Die Fumarsäure wird mindestens zur Hälfte mit Ammonium-Hydroxid ($NH_4OH$) neutralisiert.

Damit liegt auch die hergestellte L-Äpfelsäure in der Fermentationsflüssigkeit in Form des entsprechenden Salzes vor. In einer bevorzugten Ausführungsform wird zum Neutralisieren der Fumarsäure nur $NH_4OH$ eingesetzt. In einer anderen Ausführungsform wird zum Neutralisieren $NH_4OH$ und Natrium-Hydroxid (NaOH) im molaren Verhältnis 2 zu 1 eingesetzt. Die Menge der vorgelegten neutralisierten Fumarsäure kann größer als die Menge sein, die der Sättigungskonzentration entspricht; in diesem Fall liegt das Fumarat zunächst teilweise in ungelöster Form vor. Die Fumarsäure kann auch portionsweise in den Fermentationsbehälter während der laufenden Fermentation gegeben werden; dann kann man ohne zeitweise auftretendes ungelöstes Fumarat große Konzentrationen von L-Äpfelsäure erreichen.

Die biotechnische Umsetzung kann mittels Mikroorganismen, bevorzugt mit Pilzen, oder mittels Enzymen durchgeführt werden. Die Mikroorganismen können frei beweglich oder fixiert sein; die Enzyme können nicht fixiert oder fixiert sein.

Die Pilze stammen bevorzugt aus einer der Gattungen Aspergillus, Penicillium, Paecilomyces, Taphrina, Helminthosporium, Pythium, Fusarium, Hyphopichia, insbesondere aus den Arten Aspergillus wentii, Penicillium nalgiovensis, Fusarium oxysporum, Hyphopichia burtoni. Als Enzym kann die Fumarase verwendet werden; das Enzym wird aus Mikroorganismen gewonnen, die sich zur Umwandlung von Fumarat in L-Malat eignen.

Zur Durchführung des Verfahrens wird eine wäßrige Lösung oder Suspension der neutralisierten Fumarsäure hergestellt, zu der die separat gezüchtete und aus der Kulturflüssigkeit abgetrennte Zellmasse zugegeben wird. Die Konzentration der Zellmasse - berechnet als Trockenmasse - beträgt von 0,5 bis 50 g pro Liter der fumarathaltigen Fermentationsflüssigkeit. Das Fumarat wird 6 bis 48 Stunden lang bei 20 bis 60° C unter nicht-aseptischen Bedingungen fermentiert. Die Zellmasse und die L-Äpfelsäure werden nach bekannten Verfahren aus der Fermentationsflüssigkeit abgetrennt.

Frisch gezüchtete und aus der Kulturflüssigkeit abgetrennte Zellmasse kann mehrmals - bis zu dreimal, bevorzugt zweimal - in einer jeweils neu angesetzten Fumarat-Lösung oder -Suspension zur Fermentation verwendet werden.

Wegen der Fähigkeit bestimmter Mikroorganismen, auch oder gar bevorzugt in Gegenwart hoher $NH_4$-Konzentrationen Fumarsäure in L-Äpfelsäure zu überführen, ergibt sich die Möglichkeit, die Fumarsäure-Konzentration zu Beginn der Fermentation und entsprechend die L-Äpfelsäure-Konzentration zum Zeitpunkt der Ernte gegenüber den bisher bekannten Verfahren wesentlich zu erhöhen, da sich $NH_4$-Fumarat in Wasser bedeutend besser löst als Natrium- oder Kalium-Fumarat.

Die Möglichkeit, $NH_4$-Fumarat als Substrat für die Herstellung von L-Äpfelsäure einzusetzen, ist überraschend, denn wider Erwarten

- gibt es Pilze, die eine derart hohe $NH_4$-Konzentration vertragen, wie sie z. B. einer 1,8-normalen $NH_4$-Fumarat-Lösung entspricht,
- wird bei dem erfindungsgemäßen Verfahren $NH_4$-Fumarat weder auf direktem Weg (Anlagern von $NH_3$ an Fumarsäure) noch auf dem indirekten Weg (Aminierung von Oxalessigsäure, die im Metabolismus aus Fumarsäure über L-Äpfelsäure entsteht) in L-Asparaginsäure umgewandelt,
- beschleunigen $NH_4$-Ionen die biotechnische Umwandlung von Fumarat in L-Malat,
- werden bestimmte Pilze, die Natrium- und/oder Kalium-Fumarat gar nicht oder vergleichsweise langsam in das entsprechende L-Malat umwandeln, gute L-Malat-Produzenten, wenn ihnen die Fumarsäure als $NH_4$-Salz dargeboten wird.

Die für das erfindungsgemäße Verfahren geeigneten Pilze können mittels folgendem Screening-Verfahren aufgefunden werden.

Auf einem durch Agar-Agar verfestigten wäßrigen Nährboden, der neben einer Kohlenstoffquelle - z. B. Zucker - und anderen für das Wachstum erforderlichen Nährstoffen $NH_4$-Fumarat in einer Konzentration enthält, die oberhalb des Löslichkeitsproduktes des $NH_4$-Fumarats liegt, wodurch der Nährboden wegen des ungelösten Fumarats getrübt wird, werden Pilze aufgeimpft oder mit Aufschlämmungen von Bodenproben aufgebracht. Auf diesem Nährboden können nur Pilze wachsen, die eine hohe Fumarat-Konzentration vertragen. Da L-Malat wesentlich besser wasserlöslich ist als Fumarat, lassen sich unter den Pilzen, die auf diesem selektiven Nährboden wachsen, diejenigen, die Fumarat in L-Malat überführen, an einem klaren Hof um ihre Kolonien erkennen.

Andererseits können geeignete Pilze aus Stammsammlungen bezogen werden. Als Beispiele seien genannt:
- Aspergillus wentii CBS 121.32
- Aspergillus phoenicis DSM 62068
- Aspergillus awamori DSM 63272
- Penicillium nalgiovensis CBS 352.48
- Hyphopichia burtoni DSM 70663

Das erfindungsgemäße Verfahren kann sowohl diskontinuierlich als auch kontinuierlich durchgeführt werden. Die Zellen können z. B. im Fermentationsbehälter zurückgehalten werden, während die Fermentationsflüssigkeit kontinuierlich abgezogen und auf L-Äpfelsäure aufgearbeitet wird, oder die Zellen werden mit der Fermentationsflüssigkeit kontinuierlich abgezogen und nach Abtrennen von der Flüssigkeit in den Behälter zurückgeführt.

Die biotechnische Umsetzung von $NH_4$-Fumarat verläuft schneller als die Umsetzung von z. B. Natrium(Na)-Fumarat. Damit lassen sich bei gleich langer Fermentationszeit größere Konzentrationen an L-Äpfelsäure zum Zeitpunkt der Ernte errrichen.

Die Geschwindigkeit der biotechnischen Umsetzung von Fumarsäure in L-Äpfelsäure bei Einsatz von $NH_4$-Fumarat und Na-Fumarat wurde im Schüttelkolben unter folgenden Bedingungen bestimmt:

125 g/l Fumarsäure, neutralisiert mit $NH_4OH$ bzw. NaOH; Temperatur 27°C; pH-Wert 8,0; Inkubationszeit 24 Stunden; Zellmasse 1 g/l (berechnet als Trockenmasse); Zellmasse gewonnen aus einer Vorkultur mit 10 g Zucker pro Liter und 5 g Na-Fumarat pro Liter (zwecks Adaptation der Pilze).

Die Konzentration der L-Äpfelsäure in g/l zum Zeitpunkt der Ernte beträgt beispielsweise

| bei Verwendung des Pilz-stammes | ausgehend von 125 g/l Fumarsäure | |
|---|---|---|
| | als $NH_4$-Salz | als Na-Salz |
| Fusarium oxysporium | 116 | 0 |
| Aspergillus wentii | 107 | 74 |
| Aspergillus phoenicis | 112 | 23 |
| Aspergillus awamori | 119 | 66 |
| Aspergillus versicolor | 122 | 35 |
| Aspergillus chrysogenum | 115 | 23 |
| Aspergillus niger | 106 | 20 |
| Penicillium nalgiovensis | 105 | 44 |
| Hyphopichia burtoni | 104 | 27 |

Die Umwandlungsgeschwindigkeit von $NH_4$-Fumarat in L-Malat ist also generell größer und mit einigen Pilz-Stämmen sehr viel größer als die Umwandlungsgeschwindigkeit von Na-Fumarat in L-Malat.

Nicht nur die Umwandlungsgeschwindigkeit ist bei $NH_4$-Fumarat größer als bei anderen Fumaraten, sondern auch die maximal erreichbare Endkonzentration an L-Äpfelsaure zum Zeitpunkt der Ernte. Beim Neutralisieren mit NaOH lassen sich maximal ca. 170 g/l Fumarsäure in Lösung bringen, beim Neutralisieren mit $NH_4OH$ dagegen maximal 210 g/l. Da die Konzentration der L-Äpfelsäure zum Zeitpunkt der Ernte in etwa der vorgelegten Konzentration der Fumarsäure entspricht, können beim Neutralisieren mit $NH_4OH$ ca. 210 g/l L-Äpfelsäure gegenüher ca. 170 g/l beim Neutralisieren mit NaOH erreicht werden.

Bei Mischsalzen läßt sich die in Lösung zubringende Fumarsäure-Menge und damit die L-Äpfelsäure-Menge zum Zeitpunkt der Ernte weiter steigern. Wenn die Fumarsäure mit $NH_4OH$ und NaOH im molaren Verhältnis von 2 zu 1 neutralisiert wird, lassen sich ca. 240 g Fumarsäure pro Liter in Lösung bringen, womit man eine L-Äpfelsäure-Konzentration von ca. 240 g/l zum Zeitpunkt der Ernte erreicht.

Wegen der großen Umwandlungsgeschwindigkeit des Fumarats bei großen $NH_4$-Konzentrationen braucht die vorgelegte Fumaratmenge vor Beginn der Umwandlung gar nicht vollständig in Lösung gegangen zu sein. Damit kann man in die Nähe der Sättigungskonzentration des L-Malats kommen.

Legt man beispielsweise 300 g Fumarsäure pro Liter vor und stellt den pH-Wert der Suspension mit $NH_4OH$ auf 8 ein, dann liegt ein Teil des $NH_4$-Fumarats ungelöst im Ansatz vor. Mit fortschreitender Umsetzung des gelösten Fumarats in L-Malat geht auch das zunächst nicht gelöste Fumarat in Lösung und wird der Umsetzung

4

in L-Malat zugänglich. Zum Zeitpunkt der Ernte erreicht man eine L-Malat-Konzentration von ca. 300 g/l (berechnet als L-Äpfelsäure).

Die biotechnische Herstellung von L-Äpfelsäure aus Fumarsäure gemäß dieser Erfindung ist ein zweistufiger Prozeß.

In der Stufe 1 wird unter Verbrauch von Nährstoffen unter aseptischen Bedingungen die Zellmasse nach bekannten Verfahren gezüchtet. Die Kulturflüssigkeit enthält keine oder nur eine geringe Menge an neutralisierter Fumarsäure. Falls Fumarsäure zugegeben wird, dient diese zur Adaptation der Mikroorganismen.

Ist während der Züchtung keine oder fast keine Fumarsäure vorhanden, wachsen die Pilze bevorzugt pelletförmig und sind leicht abtrennbar. Bei Züchtung der Pilze in Fumarsäure-haltiger Kulturflüssigkeit wachsen die Pilze bevorzugt fadenförmig und sind schwer abtrennbar.

Wegen der in Stufe 1 notwendigen Sterilisierung der Kulturflüssigkeit wird die gegebenenfalls zugesetzte Fumarsäure in dieser Stufe bevorzugt mit NaOH oder Kalium(K)-Hydroxid neutralisiert. $NH_4$-Fumarat kann sich in der Hitze bereits teilweise verändern. Bei chemischem Sterilisieren oder beim Sterilisieren durch Filtrieren kann die Fumarsäure auch in Stufe 1 mit $NH_4OH$ neutralisiert werden.

In Stufe 2 wird Fumarat in L-Malat biotechnisch umgesetzt durch die in Stufe 1 gezüchtete und aus der Kulturflüssigkeit abgetrennte Zellmasse. Für Stufe 2 sind keine aseptischen Bedingungen erforderlich. Die Fermentationsflüssigkeit enthält zu Beginn der Umsetzung das Fumarat in hochkonzentrierter Form.

Die Züchtung der Zellmasse in Stufe 1 läuft beispielsweise wie folgt ab:
Die Nährlösung enthält pro Liter folgende Nährstoffe

```
30    g  Saccharose
2     g  Di-Ammonium-Hydrogenphosphat (NH4)2HPO4
3     g  Ammoniumsulfat (NH4)2SO4
0,9   g  Magnesiumsulfat MgSO4·7H2O
2     g  Kaliumchlorid KCl
30    mg Eisen-III-chlorid FeCl3·6H2O
```

Zum Adaptieren der Mikroorganismen können bis 30 g/l - vorzugsweise bis 5 g/l - Fumarat zugegeben werden. Falls die Mikroorganismen zum Wachsen noch Wirkstoffe benötigen (z. B. Vitamine), wird die Nährlösung noch mit diesen Wirkstoffen versetzt.

Die Nährlösung wird auf einen pH-Wert von 2 bis 9 - vorzugsweise von 3 bis 6 - eingestellt und sterilisiert. Die zum Wachsen des Pilzes erforderlichen Kohlenstoff-Verbindungen werden entweder zusammen mit den anorganischen Nährstoffen sterilisiert oder - z. B. zur Vermeidung von Karamelisierungserscheinungen bei Zuckern - separat davon. Für das Pilzwachstum sind außer Saccharose noch Maismehl, Glucose, Glycerin, n-Paraffin, Ethanol und andere kohlenstoffhaltige Verbindungen geeignet.

Die sterilisierte Nährlösung wird mit Inoculum eines für die biotechnische Umsetzung von Fumarsäure in L-Äpfelsäure geeigneten Pilzes in einer Menge von 1 bis 20 Vol.-% - vorzugsweise 3 bis 10 % - beimpft. Die Zellmasse wird unter kontrollierten Bedingungen hinsichtlich Temperatur, Belüftung und pH-Wert in 1 bis 3 Tagen unter Verbrauch der zugesetzten Kohlenstoff-Verbindung gezüchtet. Die Zuchtungstemperatur liegt bei 20 bis 50° C, bevorzugt bei 25 bis 35° C. Der pH-Wert wird während der Züchtung durch Einspeisen von Lauge konstant gehalten.

Nach Beendigung der Züchtung in Stufe 1 wird die Kulturflüssigkeit durch Filtrieren oder eine andere bekannte Trennoperation für Flüssigkeit und Feststoff in Kulturfiltrat und Zellmasse getrennt. Die Zellmasse wird zur biotechnischen Umwandlung in Stufe 2 eingesetzt.

Die biotechnische Umwandlung von Fumarat in L-Malat in Stufe 2 läuft beispielsweise wie folgt ab:
Die Zellmasse aus Stufe 1 (oder aus einer vorangegangenen Stufe 2) wird in eine Lösung oder Suspension des Fumarats überführt. Die Menge der Zellmasse (berechnet als Trockensubstanz) beträgt 0,5 bis 50 g/l des Ansatzes. Das Fumarat liegt vollständig oder überwiegend als $NH_4$-Fumarat vor, eventuell zunächst teilweise in ungelöster Form. Die Fermentationsflüssigkeit wird in einem Behälter schwach gerührt und belüftet, wobei die Pilz-Zellen das Fumarat in L-Malat umsetzen. Die Temperatur beträgt 20 bis 60° C, vorzugsweise 30 bis 45° C, der pH-Wert ist 6 bis 10, vorzugsweise 7 bis 9. Nach 6 bis 48 Stunden ist die Umsetzung auch bei großer Fumarat-Konzentration beendet. Die Menge der L-Äpfelsäure ist etwa gleich der ursprünglichen Menge der Fumarsäure.

Der in Stufe 2 verwendete Behälter kann sehr einfach gebaut sein, da keine Vorrichtungen zum Sterilisieren oder zum Erzielen einer hohen Sauerstoff-Transferrate nötig sind. Es genügt ein einfach zu reinigender thermostatisierbarer Behälter mit Vorrichtungen zum Rühren und Belüften. Schwaches Rühren ist empfehlenswert, um das Sedimentieren der Zellmasse zu verhindern; schwaches Belüften ist empfehlenswert, um die Zellen mit dem für die Erhaltungsatmung nötigen Sauerstoff zu versorgen.

Das erfindungsgemäße Verfahren hat folgende Vorteile:
- $NH_4$-Fumarat wird schneller umgewandelt als andere Fumarate, wodurch die Raum-Zeit-Ausbeute verbessert wird.
- Die Konzentration der L-Äpfelsäure zum Zeitpunkt der Ernte ist größer als bei anderen bekannten Verfahren, was die wirtschaftliche Aufarbeitung ermöglicht.
- Bei der Fermentation fällt hochkonzentrierte L-Äpfelsäure in Form ihres $NH_4$-Salzes in einer Flüssigkeit an, die außer diesem $NH_4$-Malat praktisch nur noch Reste nicht umgesetzten $NH_4$-Fumarats und Zellmasse enthält,

5

was die Aufarbeitung erheblich erleichtert.

- Das Fumarat wird mit hoher Ausbeute in L-Malat umgesetzt; die Menge der produzierten L-Äpfelsäure ist annähernd genau so groß wie die Menge der vorgelegten Fumarsäure.

- Die biotechnische Umwandlung erfolgt in einem einfach gebauten Behälter, der keine Vorrichtungen zum Erreichen und Einhalten aseptischer Bedingungen und zum Erzielen einer hohen Sauerstoff-Transferrate braucht und damit wirtschaftlich herstellbar und zu betreiben ist.

- Es entsteht keine D-Äpfelsäure.

- L-Äpfelsäure wird in einer Flüssigkeit erhalten, die deren Aufarbeitung zu einer für Nahrungsmittel und Pharmazeutika geeigneten Qualität sehr nahe kommt, z. B. hinsichtlich der Abwesenheit von LPS-Toxin und Bernsteinsäure.

- Die abgetrennte Zellmasse kann mehrfach verwendet werden.

- Die Fumarsäure wird ausschließlich unter optimalen Fermentationsbedingungen in L-Äpfelsäure umgewandelt; die für den Pilz optimalen Wachstumsbedingungen brauchen dabei nicht beachtet zu werden.

Die Erfindung wird anhand der folgenden Beispiele erläutert, ohne auf diese beschränkt zu sein.

**Züchten von Zellmasse**

**Beispiel A**

Zum Züchten von Zellmasse in Stufe 1 wird eine Nährlösung angesetzt, die pro Liter Trinkwasser

| | | |
|---|---|---|
| 30 | g | Saccharose |
| 3 | g | $(NH_4)_2SO_4$ |
| 2 | g | $(NH_4)_2HPO_4$ |
| 0,9 | g | $MgSO_4 \cdot 7H_2O$ |
| 2 | g | KCl |
| 30 | mg | $FeCl_3 \cdot 6H_2O$ |
| 10 | g | Na-Fumarat |

enthält. Ein Bio-Reaktor mit 8 Liter Arbeitsvolumen wird mit 7,2 Liter dieser Nährlösung beschickt, sterilisiert und mit 0,8 Liter Inoculum des Pilzes Aspergillus wentii angeimpft. Die Züchtungsbedingungen sind:

- Belüften mit 0,5 Liter Luft pro Liter Arbeitsvolumen des Bio-Reaktors und Minute (0,5 vvm)

- Rührerdrehzahl 500 Umdrehungen pro Minute (Blattrührer in Kombination mit einem Leitrohr)

- Temperatur 30° C

- pH-Wert 7,0 (wird durch Einspeisen von NaOH-Lösung mittels Steuerung über pH-Elektrode konstant gehalten)

Nach 48 Stunden sind 11,2 g Zellmasse (berechnet als Trockenmasse) pro Liter Kulturflüssigkeit entstanden, die abfiltriert und gewaschen werden.

**Beispiel B**

Zellmasse des Pilzes Aspergillus awamori wird analog zu Beispiel A mit folgenden Varianten gezüchtet:
- die Nährlösung enthält kein Fumarat
- der pH-Wert wird auf 6,0 eingestellt und konstant gehalten

**Beispiel C**

Zellmasse des Pilzes Penicillium nalgiovensis wird analog zu Beispiel A gezüchtet.

**Beispiel D**

Zellmasse des Pilzes Hyphopichia burtoni wird analog zu Beispiel A gezüchtet, wobei die Nährlösung statt 10 g/l Na-Fumarat 30 g/l Na-Fumarat enthält.

**Beispiel E**

Zellmasse des Pilzes Fusarium oxysporium wird analog zu Beispiel A gezüchtet.

## Biotechnische Umwandlung von Fumarsäure in L-Äpfelsäure

**Beispiel 1**

Die aus den 8 Litern Kulturflüssigkeit des Beispiels A erhaltene Zellmasse wird in 16 Liter einer 210 g/l enthaltenden Fumarsäure-Lösung (neutralisiert mit $NH_4OH$; pH = 8,0) suspendiert und in einem schwach gerührten und belüfteten Behälter bei 32°C unter nicht-aseptischen Bedingungen inkubiert. Nach 24 Stunden Inkubation sind 213 g L-Äpfelsäure pro Liter Fermentationsflüssigkeit entstanden.

**Beispiel 2**

Nach Beispiel A gezüchtete Zellmasse wird in 16 Liter einer 240 g/l enthaltenden Fumarsäure-Lösung (neutralisiert mit $NH_4OH$ und NaOH im molaren Verhältnis 2 zu 1) suspendiert. Nach 24 Stunden Inkubation sind 235 g L-Äpfelsäure pro Liter Fermentationsflüssigkeit entstanden.

**Beispiel 3**

Nach Beispiel A gezüchtete Zellmasse wird in 16 Liter einer 320 g/l enthaltenden Fumarsäure-Lösung/Suspension (neutralisiert mit $NH_4OH$) suspendiert. Nach 36 Stunden Inkubation sind 328 g L-Äpfelsäure pro Liter Fermentationsflüssigkeit entstanden.

**Beispiel 4**

Nach Beispiel A gezüchtete Zellmasse wird in 8 Liter einer 240 g/l enthaltenden Fumarsäure-Lösung (neutralisiert mit $NH_4OH$ und NaOH im molaren Verhältnis 2 zu 1) suspendiert. Nach 12 Stunden Inkubation sind 237 g L-Äpfelsäure pro Liter Fermentationsflüssigkeit entstanden.

**Beispiel 5**

Die aus der Fermentationsflüssigkeit von Beispiel 1 abgetrennte Zellmasse wird erneut in 16 Liter einer 210 g/l enthaltenden Fumarsäure-Lösung (neutralisiert mit $NH_4OH$; pH = 8,0) suspendiert; diese Zellmasse wird also zweimal zur biotechnischen Umsetzung von Fumarat in L-Malat eingesetzt. Nach 36 Stunden Inkubation sind 198 g L-Äpfelsäure pro Liter Fermentationsflüssigkeit entstanden.

**Beispiel 6**

Nach Beispiel B gezüchtete Zellmasse wird in einem Behälter mit 300 Liter Arbeitsvolumen in einer 210 g/l enthaltenden Fumarsäure-Lösung (neutralisiert mit $NH_4OH$; pH = 8,0) suspendiert. Die Konzentration der Zellmasse in der Fumarsäure-Lösung beträgt 30 g/l (berechnet als Trockenmasse). Nach 24 Stunden Inkubation sind 205 g L-Äpfelsäure pro Liter Fermentationsflüssigkeit entstanden.

**Beispiel 7**

Nach Beispiel E gezüchtete Zellmasse wird in einem Behälter mit 300 Liter Arbeitsvolumen in einer 210 g/l enthaltenden Fumarsäure-Lösung (neutralisiert mit $NH_4OH$; pH = 8,0) suspendiert. Die Konzentration der Zellmasse in der Fumarsäure-Lösung beträgt 30 g/l (berechnet als Trockenmasse). Nach 36 Stunden Inkubation sind 207 g L-Äpfelsäure pro Liter Fermentationsflüssigkeit entstanden.

**Beispiel 8**

Nach Beispiel C gezüchtete Zellmasse wird in 16 Liter einer 240 g/l enthaltenden Fumarsäure-Lösung (neutralisiert mit $NH_4OH$ und NaOH im molaren Verhältnis 2 zu 1) suspendiert. Die Konzentration der Zellmasse in der Fumarsäure-Lösung beträgt 6 g/l (berechnet als Trockenmasse). Nach 24 Stunden Inkubation sind 234 g L-Äpfelsäure pro Liter Fermentationsflüssigkeit entstanden.

**Beispiel 9**

Nach Beispiel D gezüchtete Zellmasse wird in 16 Liter einer 210 g/l enthaltenden Fumarsäure-Lösung (neutralisiert mit $NH_4OH$; pH = 8,5) suspendiert. Die Konzentration der Zellmasse in der Fumarsäure-Lösung beträgt 7 g/l (berechnet als Trockenmasse). Nach 36 Stunden Inkubation sind 212 g L-Äpfelsäure pro Liter Fermentationsflüssigkeit entstanden.

**Beispiel 10**

In einem Behälter wird eine $NH_4$-Fumarat-Suspension mit 400 g/l (berechnet als Fumarsäure) vorgelegt und auf pH 8,0 eingestellt. Das Fumarat liegt zunächst teilweise in ungelöster Form vor. In dieser Flüssigkeit werden 35 g/l Zellmasse (berechnet als Trockenmasse) - gezüchtet nach Beispiel A - suspendiert. Nach 36 Stunden Inkubation bei 32°C sind 378 g L-Äpfelsäure pro Liter Fermentationsflüssigkeit entstanden.

**Beispiel 11**

In einem Behälter wird eine $NH_4$-Fumarat-Lösung mit 190 g/l (berechnet als Fumarsäure) vorgelegt und auf pH 8,0 eingestellt. In dieser Flüssigkeit werden 29 g/l Zellmasse (berechnet als Trockenmasse) - gezüchtet nach Beispiel A suspendiert. Nach 6 Stunden Inkubation bei 34°C sind 171 g L-Äpfelsäure pro Liter Fermentationsflüssigkeit entstanden.

Bei allen Beispielen wird am Ende der Fermentation die Zellmasse und das L-Malat nach bekannten Verfahren abgetrennt. Die Zellmasse wird gegebenenfalls für eine weitere Fermentation eingesetzt, das L-Malat wird nach bekannten Verfahren zu L-Äpfelsäure aufgearbeitet.

**Patentansprüche**

1. Verfahren zum Herstellen des reinen L-Isomeren der Äpfelsäure aus neutralisierter Fumarsäure in hochkonzentrierter wäßriger Lösung durch biotechnische Umsetzung in wäßriger Phase und einer Konzentration der L-Äpfelsäure zum Zeitpunkt der Ernte von 170 bis 400 g pro Liter der Fermentationsflüssigkeit,
gekennzeichnet durch
- eine Menge der vorgelegten Fumarsäure von 170 bis 400 g pro Liter des Ansatzes,
- Neutralisieren der Fumarsäure mindestens zur Hälfte mit Ammonium-Hydroxid.
2. Verfahren nach Anspruch 1,
gekennzeichnet durch
- Neutralisieren der Fumarsäure nur mit Ammonium-Hydroxid.
3. Verfahren nach Anspruch 1,
gekennzeichnet durch
- Neutralisieren der Fumarsäure mit Ammonium-Hydroxid und Natrium-Hydroxid im molaren Verhältnis 2 zu 1.
4. Verfahren nach den Ansprüchen 1 bis 3,
gekennzeichnet durch
- eine Menge der vorgelegten neutralisierten Fumarsäure, die größer ist als die Menge, die der Sättigungskonzentration entspricht, und die zunächst teilweise ungelöst vorliegt.
5. Verfahren nach den Ansprüchen 1 bis 4,
gekennzeichnet durch
- portionsweises Zugeben von fester Fumarsäure in den Fermentationsbehälter während der laufenden Fermentation.
6. Verfahren nach den Ansprüchen 1 bis 5,
gekennzeichnet durch

- biotechnische Umsetzung mittels Mikroorganismen.

7. Verfahren nach Anspruch 6,
gekennzeichnet durch
- biotechnische Umsetzung mittels Pilzen.

8. Verfahren nach Anspruch 7,
gekennzeichnet durch
- biotechnische Umsetzung mittels Pilzen aus einer der Gattungen Aspergillus, Penicillium, Paecilomyces, Taphrina, Helminthosporium, Pythium, Fusarium, Hyphopichia.

9. Verfahren nach Anspruch 8,
gekennzeichnet durch
- biotechnische Umsetzung mittels Pilzen aus einer der Arten Aspergillus wentii, Aspergillus awamori, Penicillium nalgiovensis, Fusarium oxysporum, Hyphopichia burtoni.

10. Verfahren nach den Ansprüchen 1 bis 5,
gekennzeichnet durch
- biotechnische Umsetzung mittels Enzymen.

11. Verfahren nach Anspruch 10,
gekennzeichnet durch
- biotechnische Umsetzung mittels Fumarase.

12. Verfahren nach den Ansprüchen 1 bis 5,
gekennzeichnet durch,
- Herstellen einer wäßrigen Lösung oder Suspension der neutralisierten Fumarsäure,
- Zugeben der aus einer Züchtung von Zellmasse oder aus einer vorangegangenen Fermentation abgetrennten Zellmasse zu der Fumarsäure-Lösung oder -Suspension,
- Konzentration der Zellmasse von 0,5 bis 50 g/l (berechnet als Trockenmasse) in der fumarathaltigen Fermentationsflüssigkeit,
- biotechnische Umsetzung während 6 bis 48 Stunden bei 20 bis 60°C,
- Abtrennen der Zellmasse und der L-Äpfelsäure aus der Fermentationsflüssigkeit.

13. Verfahren nach Anspruch 12,
gekennzeichnet durch
- bis zu dreimaligem Verwenden der abgetrennten Zellmasse in einer jeweils neu angesetzten Fumarat-Lösung oder -Suspension.

14. Verfahren nach den Ansprüchen 12 und 13,
gekennzeichnet durch
- biotechnische Umsetzung unter nicht-aseptischen Bedingungen.

## Claims

1. A process for the preparation of the pure L isomer of malic acid from neutralized fumaric acid in highly concentrated aqueous solution by biotechnical conversion in aqueous phase and a concentration of L-malic acid at the time of harvesting of 170 to 400 g per litre of the fermentation liquid,
characterized by
an amount of initially introduced fumaric acid of 170 to 400 g per litre of the mixture, neutralization of the fumaric acid to the extent of at least one half with ammonium hydroxide.

2. A process according to claim 1,
characterized by
neutralization of the fumaric acid only with ammonium hydroxide.

3. A process according to claim 1,
characterized by
neutralization of the fumaric acid with ammonium hydroxide and sodium hydroxide in the molar ratio 2 to 1.

4. A process according to any of claims 1 to 3,
characterized by
an amount of initially introduced neutralized fumaric acid which is larger than the amount corresponding to the saturation concentration and which initially is partially in undissolved form.

5. A process according to any of claims 1 to 4,
characterized by
addition of solid fumaric acid in portions to the fermentation vessel while the fermentation is in progress.

6. A process according to any of claims 1 to 5,
characterized by
biotechnical conversion by means of microorganisms.

7. A process according to claim 6,
characterized by
biotechnical conversion by means of fungi.

8. A process according to claim 7,

0 175 007

characterized by

biotechnical conversion by means of fungi from one of the genera Aspergillus, Penicillium, Paecilomyces, Taphrina, Helminthosporium, Pythium, Fusarium, Hyphopichia.

9. A process according to claim 8,

characterized by

biotechnical conversion by means of fungi from one of the species Aspergillus wentii, Aspergillus awamori, Penicillium nalgiovensis, Fusarium oxysporium, Hyphopichia burtoni.

10. A process according to any of claims 1 to 5,

characterized by

biotechnical conversion by means of enzymes.

11. A process according to claim 10,

characterized by

biotechnical conversion by means of fumarase.

12. A process according to any of claims 1 to 5,

characterized by

preparation of an aqueous solution or suspension of the neutralized fumaric acid, addition of a cell mass which has been removed from a cultivation of cell mass or from a previous fermentation to the solution or suspension of fumaric acid, concentration of the cell mass from 0.5 g/l to 50 g/l (calculated as dry matter) in the fumarate-containing fermentation liquid, biotechnical conversion at 20 to 60°C for 6 to 48 hours, removal of the cell mass and the L-malic acid from the fermentation liquid.

13. A process according to claim 12,

characterized by

the removed cell mass being used up to three times in a solution or suspension of fumarate which is prepared anew each time.

14. A process according to claim 12 or 13,

characterized by

biotechnical conversion under non-aseptic conditions.


## Revendications

1. Procédé de préparation à l'état pur de l'isomère lévogyre de l'acide malique à partir d'acide fumarique neutralisé en solution aqueuse hautement concentrée, par réaction biotechnique en phase aqueuse et à une concentration d'acide L-malique au moment de la récolte de 170 à 400 g par litre du liquide de fermentation, caractérisé par le fait

- qu'on met préalablement en place l'acide fumarique à raison d'une quantité de 170 à 400 g par litre de la préparation,

- on neutralise l'acide fumarique au moins pour moitié avec de l'hydroxyde d'ammonium.

2. Procédé selon la revendication 1,

caractérisé par le fait que l'on neutralise l'acide fumarique uniquement avec de l'hydroxyde d'ammonium.

3. Procédé selon la revendication 1,

caractérisé par le fait que l'on neutralise l'acide fumarique avec de l'hydroxyde d'ammonium et de l'hydroxyde de sodium dans un rapport molaire de 2 à 1.

4. Procédé selon les revendications 1 à 3,

caractérisé par le fait que l'on utilise l'acide fumarique neutralisé et préalablement mis en place dans une quantité qui est plus grande que celle correspondant à la concentration de saturation et qui est d'abord présente en partie à l'état non-dissous.

5. Procédé selon les revendications 1 à 4,

caractérisé par le fait que l'on ajoute par portions de l'acide fumarique solide dans le récipient de fermentation pendant le déroulement de la fermentation,

6. Procédé selon les revendications 1 à 5,

caractérisé par le fait que l'on effectue la réaction biotechnique à l'aide de micro-organismes.

7. Procédé selon la revendication 6,

caractérisé par le fait que l'on effectue la réaction biotechnique à l'aide de champignons.

8. Procédé selon la revendication 7,

caractérisé par le fait que l'on effectue la réaction biotechnique à l'aide de champignons de l'une des espèces suivantes: Aspergillus, Penicillium, Paecilomyces, Taphrina, Helminthosporium, Pythium, Fusarium, Hyphopichia.

9. Procédé selon la revendication 8,

caractérisé par le fait que l'on effectue la réaction biotechnique à l'aide de champignons de l'une des categories suivantes: Aspergillus wentii, Aspergillus awamori, Penicillium nalgiovensis, Fusarium oxysporium, Hyphopichia burtoni.

10. Procédé selon les revendications 1 à 5,

caractérisé par le fait que l'on effectue la réaction biotechnique à l'aide d'enzymes.

11. Procédé selon la revendication 10,
caractérisé par le fait que l'on effectue la réaction biotechnique à l'aide de fumarase.

12. Procédé selon les revendications 1 à 5,
caractérisé par le fait

- que l'on prépare une solution ou suspension aqueuse de l'acide fumarique neutralisé,
- que l'on ajoute, à la solution ou suspension d'acide fumarique, la masse cellulaire séparée provenant d'une culture de masse cellulaire ou d'une fermentation préalable.
- que l'on concentre la masse cellulaire de 0,5 à 50 g par litre (calculée en tant que masse sèche) dans le liquide de fermentation contenant du fumarate,.
- que l'on effectue la réaction biotechnique pendant 6 à 48 heures à une température de 20 à 60°C,
- que l'on sépare la masse cellulaire et l'acide L-malique du liquide de fermentation.

13. Procédé selon la revendication 12,
caractérisé par le fait que l'on utilise jusqu'à trois fois la masse cellulaire séparée, dans une solution ou suspension de fumarate renouvelée à chaque fois.

14. Procédé selon les revendications 12 et 13,
caractérisé par le fait que l'on effectue la réaction biotechnique dans des conditions non-aseptiques.